# EUROPEAN PATENT APPLICATION

(11) **EP 0 773 292 A1**
(43) Date of publication of application: **14.05.1997**
(21) Application number: 96117118.8
(22) Date of filing: 25.10.1996
(51) Int. Cl.: C12N 15/12, C12N 5/10

(54) **Immortalised embryonic hippocampal cells and their use**

(30) Priority: 07.11.1995 EP 95117502
(71) Applicant: BAYER AG, 51368 Leverkusen (DE)
(72) Inventor: Antonicek, Horst-Peter, Dr., 51467 Bergisch Gladbach (DE); Flocke, Karola, Dr., 42799 Leichlingen (DE); Müller, Thomas, Dr., 53113 Bonn (DE); Friedl, Arno, Dr., 51427 Bergisch Gladbach (DE)

(57) **Abstract**

This invention describes immortalised murine cell lines generated by infection of embryonic hippocampal cells with specific oncogenes, which express stable properties including receptors for neurotransmitters including glutamate receptors.

## Description

### 1. Field of the invention

This invention describes immortalised murine cell lines generated by infection of embryonic hippocampal cells with retroviruses encoding specific oncogenes, which express stable properties including receptors for neurotransmitters.

### II. Description of the Related Art

In order to develop neuroactive drugs for treating a wide range of neurological disorders such as stroke, the availability of fast reliable screening methods is of vital importance. These facilitate a quick selection of new compounds which then can be further tested in more complex *in vitro* and *in vivo* models. In many neurological disorders receptors for neurotransmitters such as glutamate may be involved in the final pathway leading to neuronal cell death; in some instances overstimulation of such receptors may lead to an excessive rise in intracellular calcium levels which kill the cell. Thus, the development of methods to interfere with this destructive pathway, such as receptor antagonists, may well be therapeutically important. To screen such substances two major experimental approaches are generally applied: 1) the application of compounds to the appropriate neural cells either in slices of intact tissue or in cell culture or 2) application to non-neural cells such as fibroblasts or oocytes which have been induced to express the receptors or channels of interest via transfection of the appropriate genes. In both instances electrophysiological measurements on single cells are generally used to test the reaction to the applied substances. The first method, although being closest to the *in vivo* situation, suffers from the disadvantage that the use of primary cultures is tedious and time consuming and makes the screening of large numbers of potentially interesting compounds difficult. The second approach, even if stable transfectants are available, suffers from the disadvantage that the transfected cells are not of neural origin. The interaction of the transfected gene products with signal transduction pathways inside the cell may not mimic exactly the physiological situation. Additionally, the receptors of interest (most of which are composed of multiple subunits) have to have been cloned.

To overcome these problems, the availability of stable immortalised neural cell lines expressing the receptors and channels of interest and serving as a permanent source of material would be very advantageous. Such lines could be kept as frozen stocks, thawed and reestablished in cell culture as new batches of potential neuroactive substances became available. In addition, large numbers of cells could be obtained facilitating screening using biochemical methods. Although many publications have described the generation of immortalised neural cell lines, the stable expression of neurotransmitter receptors by a high proportion of cells in such cell lines as we describe in this invention has not been achieved.

### Background of the present invention

Some cell lines with neuronal or bipotential properties have been described by several groups. Bartlett et al. (1988, Proc. Natl. Acad. Sci. USA 85:3255-3259) generated immortalised cell lines from embryonic mouse neuroepithelium by infecting the primary cells with a retrovirus containing the *c-myc* oncogene. The cell lines showed a stable morphology and antigen expression compatible with that known for normal neuroepithelium. Differentiation could be induced by addition of acidic or basic fibroblast growth factor to the culture medium. The cells differentiated into either astrocytes expressing glial fibrillary acidic protein, or neurons positive for the A2B5 marker and containing neurofilaments. Birren and Anderson (1990, Neuron 4:189-201) immortalised sympathoadrenal progenitors using a *v-myc*-containing retrovirus. In the presence of NGF and FGF a small percentage of the cells differentiated to NGF-dependent postmitotic neurons. Ryder et al. (1989, J. Neurobiol. 21:356-375) generated neural cell lines using a retroviral vector expressing the *v-myc* oncogene and infecting mitotic progenitor cells of postnatal mouse olfactory bulb and cerebellum or postnatal rat cerebral cortex as target cells. The infection of the last two tissues resulted in multipotential clonal lines which spontaneously exhibited neuronal and glial phenotypes and the cerebellar lines differentiated into neurons and glia when transplanted in vivo (Snyder et al., 1992, Cell 68:33-51). The infection of the cerebral cortex resulted in mortal clones of glial character. Renfranz et al. (1991, Cell 66:713-729) described the establishment of a nestin-positive cell line originating from embryonic precursor cells of rat hippocampus. The cells were immortalised using a retrovirus encoding a temperature sensitive allele of *SV 40 T* antigen containing retrovirus. The cells were implanted into neonatal hippocampus and cerebellum and exhibited morphologies typical for neurons and glial cells. The following two groups described cell lines expressing transmitter receptors: Evrard et al. (1990, Proc. Natl. Sci. USA 87:3062-3066) immortalised newborn mouse striatum using of *SV 40 T* oncogene-expressing retroviruses. They obtained bipotential and plastic glio-neuronal precursors. Depending on the culture conditions the cells expressed either glial fibrillary acidic protein or neurofilaments. Some cells expressed adrenergic, D1 and D2 dopaminergic, muscarinic, and 5-hydroxytryptamine type 2 serotonergic receptors as judged by immunofluorescence analysis and measurements of inositol phosphate increase in the presence of neurotransmitters. Morimoto et al. (1990, Proc. Natl. Acad. Sci. 87:3518-3521) identified a functional N-methyl-D-aspartate receptor on HT-4 cells by neurotransmitter uptake and secretion kinetics. HT-4 is a clonal neural line originating from mouse striatum created by immortalisation with a *SV 40 T*-containing retroviral vector.

Recently, Youkin et al. (1993, Proc. Natl. Acad. Sci.90:2174-2178) have demonstrated that the NT2 human cell line, originating from teratocarcinoma cells, expresses N-methyl-D-aspartate (NMDA) and non-NMDA glutamate receptors, as shown by electrophysiological recordings using the patch-clamp technique from single cells. However, it is unclear from the published data how many cells of the line exhibit these electrophysiological responses to application of neurotransmitter.

### Summary of the invention

The present invention describes the generation of stable immortalised neural cell lines and clones derived from them. These lines and clones express neurotransmitter receptors as detected by biochemical and imaging techniques, with similar properties to the receptors expressed by normal neurons. These cell lines and clones can be frozen and stored in liquid nitrogen and reestablished in culture without changing their properties. They are stable in culture over many months of continual passage, can be continuously propagated and express receptors for a range of neurotransmitters. In addition, they can be induced to differentiate towards a more neuronal phenotype as judged by the expression of certain antigenic and electrophysiological properties by specific treatment. The number of cells obtained which react to neurotransmitters permits the utilisation of biochemical as well as electrophysiological screening methods.

In particular, these cells represent a useful tool for screening compounds with potential neuroprotective activity. By incubating the cells with an excitotoxic agent such as glutamate for a certain period of time cell death is induced. Neuroprotection as measured by prevention of cell death may be achieved by various mechanisms. Since these cells are equipped with a variety of neurotransmitter receptors and ion channels they provide a generally applicable system with which to test compounds with different mechanisms of action.

In addition, as these cells express neuronal-type ion channels including calcium, sodium and potassium channels, they can be used to screen specific modulators of these channels, which might be useful for the treatment of a variety of CNS disorders including cerebral ischemia, mood disorders, psychosis, and cognitive deficits. They also possess various neurotransmitter receptors and are thus suitable for studying receptor-related processes. Furthermore, these cell lines can be used to study distinct stages of neuronal differentiation in the hippocampus in vitro and as an assay for factors involved in differentiation.

After analysis in vitro, the cell lines could be transplanted into animals to yield information concerning their differentiation potential in vivo. The fact that the cell lines can be genetically manipulated before transplantation permits the analysis of the effect of specific genetic changes on the behaviour of the cells in the animals. This may include a possible therapeutic potential of these manipulated cell lines such as secretion of neuroprotective or regeneration supporting factors.

Cell lines according to the present invention have been deposited on August 8th and 10th 1995 with the
DSM-DEUTSCHE SAMMLUNG
VON MIKROORGANISMEN UND
ZELLKULTUREN GmbH
Maschenroder Weg 1b
38124 Braunschweig
Germany
under designations ACC 2223, ACC 2230, ACC 2231, ACC 2232 and ACC 2233

### METHODS

### Primary culture of hippocampal neurons

Hippocampi were taken from E16-day-old-mice. The cell cultures were set up according to the method described in Dotti for the rat (Dotti et al., 1988, J. Neurosci. 8;1454-1468), after modification for the mouse as explained below. After removing the meninges the hippocampal tissue was dissociated using 0.3% trypsin for 15 minutes at 37°C. The cells were then washed three times in HBSS⁻for 5 minutes at 37°C and dissociated by trituration using fire polished pasteur pipettes of different diameters. The cells were then resuspended in MEM/HS and plated on pLL-coated glass coverslips (2-4 x 10⁴ cells per 11 mm coverslip), or petri-dishes (3-5 x 10⁵ cells per 60 mm diameter; NUNC, Denmark). After attachment of the cells (5 h to overnight) the medium was changed to N2-medium (MEM containing 25 mM NaHCO₃, 0.6% glucose, 20 nM glutamine, 1% albumin, 1mM sodium pyruvic acid, 100 µg/ml transferrin, 5µg/ml insulin, 20 nM progesterone, 100 µM putrescine, 30 nM sodium selenite) and cytosine arabinoside (Sigma, 5 x 10⁻⁶ M) was added for 2-3 days to kill proliferating glial cells. Cultures of early postnatal glial cells seeded on coverslips with wax feet were added as feeder cells. Once a week 2/3 of the medium was changed.

### Derivation of immortalised lines via infection of primary murine hippocampal cells with retroviruses expressing the v-myc gene or the egfr-neu-gene

*The producer cell line GPE-egfr-neu* (Jung et al. 1995, Eur.J.Neurosci.7:1245-1265) containing the *egfr-neu* hybrid oncogene and the neomycin gene under the control of the long terminal repeats of the Mouse Moloney Leukemia Virus and the *egfr-neu* oncogene additionally under the control of an internal Herpes Simplex Thymidine Kinase Promotor (TK), was cultured in Dulbecco's modification of Eagles medium (DMEM) containing 10% fetal calf serum. The psi2 producer cell line expressing the *v-myc* oncogene and the neomycin gene under the control of the long terminal repeats of the Mouse Moloney Leukemia Virus (Wagner et al. 1985, EMBO J. 4:663-666) was cultured in DMEM containing 10% fetal calf serum. The retrovirus containing supernatants were harvested from near confluent cell cultures after 16-18 hours. The hippocampal cells (1 x 10⁷ cells per 60 mm Petri dish) were incubated immediately after the preparation at 37°C with the retrovirus-containing supernatants for four to six hours in the presence of 6 µg/ml polybrene (Sigma, München, B.R.D) and EGF (10 ng/ml); β-FGF (1ng/ml) and NGF (10 ng/ml). Thereafter the cells were incubated for 2 days at 37°C in N2-medium containing 1% horse serum and EGF (10 ng/ml), β-FGF (1 ng/ml) and NGF (10 ng/ml) - subsequently called N2⁺-medium - before selection of infected cells for neomycin resistance in the presence of 0.3 mg/ml G418 (Sigma, München, D). The surviving cells were cultured at 37°C in SATO-medium containing 1% horse serum. Two thirds of the medium was changed every 3-4 days, EGF was added to the medium of the cells infected with the *egfr-neu* virus every 2 days.

### Cloning of the cell lines

Cells of the different established cell lines were released from petri dishes with 0.01% trypsin/0.02% EDTA and after a washing step in BME containing 10% horse serum (10 minutes for 800 rpm at 4°C) resuspended in N2⁺-medium at a very low dilution. Using an extremely fine fire polished pasteur pipette, the diluted cell suspension was seeded in a 96-well-plate such that each well contained one drop of medium containing only one cell, this was controlled under the microscope. To these wells were then added feeder cells, either thymocytes or cortical glial cells (200,000 cells per well). After two weeks in culture the proliferating cell clones were selected using N2⁺-medium containing 0.3 mg/ml G418 for 5-8 days. The cells were passaged in N2⁺-medium and aliquots frozen as a permanent stock.

### Thymocyte feeder cells

Thymocytes from 4- to 6-week-old female NMRI mice were prepared as follows: thymuses were removed and a single cell suspension was prepared, after which the cells were washed in BME medium containing 10% horse serum and then plated in 96-well-plates (NUNC, Denmark) at a density of 200,000 cells per well.

### Primary glial cells from neonatal cortex as feeder cells

Glial cells fom cortex were prepared using postnatal day 0 or 1 mice. After decapitation the cortex was taken and freed from meninges. The tissue was dissociated by two trypsination steps using as a first step 1% trypsin in Hank 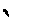 s buffered salt solution (HBSS⁻) for 15 minutes at 37°C and as a second step 0.2% trypsin, 0.02% EDTA in HBSS⁻ for 15 minutes at 37°C. Supernatant was then removed and after a washing step with HBSS⁻0.5% DNAse was added. The cells were then dissociated by trituration using fire polished pasteur pipettes of different diameters. After a washing step in Eagle 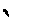 s basal medium (BME/HS) at 800 rpm for 10 minutes the pellet was resuspended in minimal essential medium (MEM/HS containing NaHCO₃ 25 mM, 20 nM glutamine, 10% horse serum, 0.6% glucose; Gibco) and plated either in pLLysin-coated petri-dishes (1-2 x 10⁷ cells per 60 mm diameter; NUNC, Denmark) or on pLL-coated (0.01%) glass coverslips (1 x 10⁴ cells per coverslip) with wax dot feet (paraffin solidification at 42-44°C; Merck).

### Induction of differentiation

For experiments in the presence of growth factors or other differentiation inducing agents, the primary cell cultures or the immortalised cell lines were cultured in the presence of one or a combination of the following: β-FGF, 1ng/ml, (Collaborative Research), EGF; 5 ng/ml or 10 ng/ml, (Collaborative Research), NGF (10 ng/ml, Collaborative Reasearch), dibutyryl cyclic adenosine monophosphate (dbcAMP, 1mM, Sigma, Heidelberg), retinoic acid (5 µM; Sigma), IL-7 (50 U/ml), TGFα (5ng/ml).

### Co-culture of primary cells and immortalized cell clones

Immortalized cells of the different cell clones were co-cultured together with either cortical glial cells or granule cells from cerebellum. The primary cells were plated in a 60 mm petri-dish as described above and the cells of the cell clones were plated on pLL-coated glass coverslips with wax feet to maintain a short distance between the primary cells and the immortalised cells.

The wax dots were prepared as follows: autoclaved paraffin was dotted on the coverslips using a fine pipette making three fine dots per coverslip. After solidation of the dots the coverslips were coated with pLL for at least 30 minutes at 37°C and washed with the appropriate medium prior to plating the cells.

### Granule cell neurons

Granule cells were prepared as described by Keilhauer et al. (1985, Nature 316:728-730). The cells enriched by a Percoll centrifugation step were finally resuspended in modified SATO medium (Trotter et al., 1989, J. Neurosci. Res. 22:369-383) containing additionally 10% horse serum, 25 mM KCl and 0.6% glucose. After 2 days of culture cytosine arabinoside (10⁻⁵ M) was added to the medium to kill any remaining non-neuronal cells. For co-culture studies, 1-2 x 10⁷ cells were plated in a petri dish (60 mm diameter; Nunc, Denmark).

### Antibodies

To determine which cell types were present in the cultures, the following antibodies were used: rabbit polyclonal antibodies against glial fibrillary acidic protein (GFAP, Dako, Hamburg, Germany); murine monoclonal antibodies against the A2B5 antigen expressed by O-2A glial precursor cells (Raff et al., 1983, Nature 303:390-396; 1984; Dev. Biol. 106:53-60), some astrocytes (Raff et al., 1983, Nature 303: 390-396; Schnitzer and Schachner 1981, J, Neuroimmunol. 1:457-470) and neurons (Eisenbarth et al., 1979, Proc. Natl. Acad. Sci. (USA) 76:4913-4917); murine monoclonal antibody against the O4 antigen recognising oligodendrocytes, a late stage of precursor cells and Schwann cells (Schachner et al., 1981, Dev. Biol. 83:328-338; Sommer and Schachner 1981, Dev. Biol. 83:311-327; Trotter and Schachner, 1989, Dev. Brain Res. 46:115-122); FITC-coupled murine monoclonal antibody against bromodeoxyuridine (Becton-Dickinson, Heidelberg, Germany); monoclonal rat L1 antibody recognising neurons and Schwann cells (Rathjen and Schachner, 1984, EMBO J. 3:1-10); mouse monoclonal antibody LB1 directed against the ganglioside GD3 recognising glial precursor cells and an early stage of neuroectodermal cells (Reynolds and Wilkin, 1988, Development 102:409-425); polyclonal antibodies against nestin (a kind gift from E. Aaku-Sarkaste; University of Heidelberg, Germany); monoclonal antibody recognizing the 160 kD form of neurofilament (Boehringer Mannheim; Germany); monoclonal antibody recognizing the early phosphorylated form of the high molecular weight kD form of neurofilament (Pennymaker et al. 1991, Exptl.Neurology 111:25-35); monoclonal antibody against vimentin (Dianova, Hamburg, Germany); monoclonal antibody recognizing the microtuble-associated protein tau (Boehringer Mannheim; Germany); monoclonal antibodies against Synaptophysin (Boehringer Mannheim; Germany); polyclonal antibodies recognizing neuron specific enolase (Dianova, Hamburg; Germany); monoclonal antibodies against the microtubule-associated-protein 2 (MAP-2; Boehringer Mannheim; Germany). Monoclonal antibodies were prepared from hybridoma culture supernatants by ammonium sulphate precipitation and dialysis against phosphate buffered saline (PBS), or were available commercially.

FITC- or TRITC-coupled anti-mouse, anti-rat, anti-goat antibodies or anti-rabbit antibodies were from DAKO, Cappel (Denkendorf, Germany) or Dianova. Staining of cultures for indirect immunfluorescence was carried out as described by Schnitzer and Schachner (1981).

### Detection of DNA synthesis in dividing cells using bromodeoxyuridine (BrdU) incorporation

The primary hippocampal cells plated on pLL-coated coverslips (1-2 x 10⁵ cells/coverslip) were incubated for 15 hr in N2⁺-medium with addition of BrdU (5µM). The cells were then washed in phosphate-buffered saline (PBS), incubated for 20 minutes with the first antibody (L1) and, after a washing step in PBS, incubated with goat anti-mouse TRITC. The cells were then fixed for 30 minutes in ice-cold 0.5% paraformaldehyde in PBS and then for 20 minutes in 4% paraformaldehyde in PBS at room temperature. After a washing step in PBS the cells were incubated for 20 minutes in 4 N HCl/0.5% Tween 20 followed by 5 minutes in 0.1 M Borax. After a further washing step in PBS the cells were incubated for 30 minutes with the FITC-coupled monoclonal antibody against BrdU. After washing in PBS the cells were examined under a fluorescence microscope.

### Test of virus production

All immortalised cell lines and clones resulting from infections with Ψ2-based vectors (v-myc) were tested periodically for the release of infectious virus carrying the inserted genes by incubating 3T3 fibroblasts (1 x 10⁷ cells/60 mm petri dish) in supernatants from the hippocampal cell lines in the presence of polybrene, followed by a selection step in G418 containing culture medium (DMEM plus 10% fetal calf serum). No G418-resistant 3T3 cells were ever observed.

### Ca²⁺-imaging procedures

**Fluorescent Probe:** Dynamic changes of intracellular calcium concentration were measured using the Fura-2 fluorescent probe (Roe et al. 1990, Cell Calcium 11:63-73). This fluorescent dye was chosen because of its selectivity, high quantum yield and extinction co-efficient. Fura-2 permeates the living cell as a acetoxymethylester and becomes trapped within the cell after hydrolysis by intracellular esterases. The calcium-sensitive free acid of Fura-2 is able to bind to calcium ions inside the cell. Fura-2 has an excitation maximum at 380 nm and the Fura2/calcium complex peaks at 340 nm. A change in intracellular calcium concentration was calculated by recording fluorescent video images at wavelengths of 340nm and 380nm excitation and 510nm emission and by dividing the grey values of the digitized video images of 380nm from 340nm.

Loading cells with Fura-2: A stock solution with 2mM Fura-2-acetoxymethylester in dimethylsulfoxide (DMSO) was diluted to 5-10 µM with serum free Minimal Essential Medium (MEM, Gibco) containing 1.5% bovine serum albumin and 5 mM calcium chloride. The cells were incubated in a Falcon 96 well plate for 45-60 minutes at 37°C. Subsequently the cells were washed twice in N-(2-Hydroxyethyl)piperazine-N'-(2-ethanesulfonic acid) (HEPES, 5mM) buffered Tyrode solution (HEPES buffered salt solution containing 130 mM NaCl, 5 mM KCl, 2 mM CaCl₂, 1 mM MgCl₂, 5mM NaHCO₃, 11 mM Glucose).

Imaging procedure: 100 µl of Tyrode buffer was added to the plate wells and the cells were sequencially exposed to light at 340 nm and 380 nm under a fluorescent microscope (Nikon Diaphot). The exposition time was 100 ms. A series of video images at 3 second intervals was acquired and stored as digitised pictures in the image boards of an image analysis computer (Leica, Quantimet 570). After the acquisition of 8 images the test substances were applied. The grey levels of the cells, which correspond to the fluorescence intensity, were measured in the recorded video images. The ratio was determined by dividing the measured grey levels of the "380 nm images" by the grey levels of the "340 nm images" either from averaged images or from individual cells. For averaged results, the grey levels were measured over the whole images.

### Second messenger procedures

Phosphoinositide hydrolysis was determined by measuring the accumulation of ³H-inositol phosphates. The cells were plated in 48-well plates (Costar) at a density of 10000 cells per well and incubated for 4-5 days at 37°C, 7% CO2. Then the cells were labeled by incubation with 4 µCi of myo-2-³H-inositol per ml medium (specific activity: 10-20 µCi/mmol: Amersham). After 24 h LiCl (10 mM final concentration) was added to the cells for 1 h. Agonists were then added at the concentrations indicated. The incubation was stopped after 1 h by replacing the medium with 200 µl ice-cold methanol. 400 µl of destilled water was added and the sample was directly applied to AG-1X8 ion exchange columns (AG-1X8 resin: BioRad). Free inositol and glycerophosphoryl-inositol were washed away with 16 ml destilled water followed by 10 ml 5 mM disodium-tetraborate, 60 mM sodium formate. Total ³H-inositol phosphates (IP1, IP2, IP3) were eluted with 7 ml of 100 mM formic acid, 1 M ammonium formate. Radioactivity of the eluted ³H-inositol phosphates was determined by liquid scintillation counting after addition of 13 ml liquid scintillation cocktail (Ultima Gold: Canberra Packard). Measurements were performed in triplicates for each experimental point. Untreated controls were set as 100% and the other values calculated in relation to this.

The columns were prepared as follows: 1 ml pipet tips were filled with one glass bead (Roth) per tip before adding 500 µl AG-1X8 resin. The resin was compressed with air and 270 µl seasand was applied. Until use they were stored at 4°C.

### Electrophysiology

Electrophysiological setup: Membrane currents were measured with the patch-clamp technique in the whole-cell recording configuration (Hamill et al. 1981, Pfluegers Arch. 391:85-100). Current signals were amplified with conventional electronics (EPC-9 amplifier, HEKA Elektronik GmbH, Lambrecht, Germany), filtered at 10kHz, and sampled at 5 kHz by an interface connected to an IBM AT-compatible computer system, which also served as a stimulus generator.

Solutions and electrodes: The standard bathing solution contained (in mM): Nacl 150, KCL 5, MgCl₂ 1.8, HEPES 5, and glucose 10. In some experiments an equimolar amount of NaCl was replaced by 5mM BaCl_{2.} Recording pipettes had resistances about 5MΩ (Hilgenberg, Malsfeld, Germany). The pipettes contained (in mM): KCL 140, CaCl₂ 0.5, EGTA 5, MgCl₂ 2, and HEPES 10. The pH was adjusted to 7.4. In some experiments the pipette solution was designed to minimize the contribution of voltage-activated currents. In this case, investigating the ligand-activated currents, the pipette solution contained (in mM): CsCl 140, EGTA 10, and HEPES 10.

Ligands were dissolved in the extracellular saline solution and applied via an U-tube application system. To record ligand-induced currents, the membrane potential was clamped to -70mV. For measurements of voltage-activated currents, cells were held at -70mV and every second the membrane was stepped through a 50 msec. de- or hyperpolarising test pulse to a potential between -170 and +20 mV.

### RESULTS

### Summary of cell lines and clones

The following cell lines and clones are included in the patent:
1.) Cell lines originating from an infection step using the v-myc oncogene expressing retroviral vector:
   HNMMCV 13.1.
   HNMMCV 14.1.
   HNMMCV 11.2.
   HNMMCV 13.1.F and HNMMCV 13.1.E are clones derived from the cell line HNMMCV 13.1.
   HNMMCV 11.2.O is a clone derived from the cell line HNMMCV 11.2.
   HNMMCV 14.1.A is a cell clone derived from the cell line HNMMCV 14.1.
2.) Cell lines originating from an infection step using the egfr-neu hybrid gene expressing retroviral vector:
   HC e/n XV
   HC e/n XVB is a clone derived from the cell line HC e/n XV.

### Establishment of primary cultures from murine hippocampus

Mice from embryonal day E16 were taken to prepare the hippocampi as described in materials and methods. The use of feeder cells enables interaction between the cells via released factors but excludes a direct cell to cell contact. With the help of this system it is possible to maintain the murine hippocampal cells for at least 21 days in culture. The cells produce first many lamellipodia, which were then reduced to some distinct processes differentiating and fasciculating after longer times in culture.

### Characterisation of the primary hippocampal cells

The hippocampal cells were analysed for the expression of various markers using immunofluorescence techniques. The following antibodies were used: GFAP, nestin, neurofilament (160 kD; phosphorylated form), MAP-2, tau, synaptophysin, LB1, L1, vimentin, O4, A2B5, Neuron-Specific Enolase. The results are summarized in Table 1.

### Immortalization and selection of the hippocampal cells

The cultures were analysed for the presence of mature cells which had developed from dividing precursor cells, by examining the incorporation of BrdU into DNA into cells which had developed in cultures 2-7 days after a 16 hour incubation with BrdU. Some GFAP-positive cells of astrocytic origin, many nestin-positive cells of precursor-like origin and a few L1-positive cells of neuronal origin were detected. Immortalised cell lines were established from the primary cultures using the various retroviral vectors containing the v-myc, or the egfr-neu oncogene according to the procedures described in material and methods. The surviving successfully immortalised cells were analysed for their marker profile and cloned as described above.

Using both the v-myc and the egfr-neu oncogene-containing vectors, several stable cell lines were generated, which could be passaged for many months. The cells of the cell lines consisted either of only flat, or of flat and bipolar cells, while the bipolar cells resembled morphologically the primary hippocampal cells two days after plating.

The cell lines originating from the v-myc and egfr-neu oncogene containing retroviral constructs were characterised for their marker expression. Results are shown in table 1 and 2. The cell lines containing the v-myc oncogene were composed of different cell types. The cell lines HNMMCV13.1, HNMMCV11.2 and HNMMCV14.1 had mainly a bipolar morphology, while only a small percentage of the cells were flat and astrocyte-like. The cell lines were investigated for their expression of the same markers as used for the primary hippocampal cells. In summary, all cell lines contained many vimentin- and nestin-positive cells and a small percentage of LB1- and GFAP-positive-cells (table 1).

The cell lines expressing the egfr-neu oncogene exhibited mainly a very flat and undifferentiated looking morphology. Almost all cells were vimentin- and nestin-positive, a relatively high percentage expressed A2B5, GFAP and LB1, and a few cells were positive for O4 and MAP-2 (table 2).

**Table I**

| antigenic profile of primary hippocampal cells and v-myc expressing cell lines and clones | | | | | | | | | | | |
|---|---|---|---|---|---|---|---|---|---|---|---|
| | Antibodies | | | | | | | | | | |
| | A2B5 | LB1 | O4 | L1 | GFAP | NS | VIM | NF-P | tau | Synaptp. | MAP-2 |
| | | | | | | | | | | | |

| Primary Cells | | | | | | | | | | | |
|---|---|---|---|---|---|---|---|---|---|---|---|
| 4 DIV | (+) | (+) | + | +++ | + | + | +++ | ++ | ++ | ++ | + |

| cell lines | | | | | | | | | | | |
|---|---|---|---|---|---|---|---|---|---|---|---|
| 13.1 | - | ++ | - | - | - | +++ | +++ | - | - | - | - |
| 14.1 | - | ++ | - | - | + | +++ | +++ | | | | - |
| 11.2 | + | +++ | - | - | + | +++ | - | - | - | - | - |
| 4.2 | - | | - | - | ++ | +++ | - | | | | |

| cell clones | | | | | | | | | | | |
|---|---|---|---|---|---|---|---|---|---|---|---|
| 13.1.E | | +++ | - | - | + | +++ | +++ | - | - | - | - |
| 13.1.F | | + | - | - | + | +++ | +++ | - | - | - | - |
| 11.2.N | | - | - | - | - | +++ | +++ | - | - | - | - |
| 11.2.O | | ++ | - | - | ++ | ++ | +++ | - | - | - | - |

**Table 2**

| antigenic profile of egfr-neu virus expressing cell clone HC e/n XV B + EGF | | | | | | | | |
|---|---|---|---|---|---|---|---|---|
| | Antibodies | | | | | | | |
| | A2B5 | LB1 | O4 | L1 | GFAP | NS | VIM | MAP-2 |
| cell clone: | | | | | | | | |
| HC e/n XV B | ++ | ++ | + | - | + | +++ | +++ | + |

### Cell clones

The cell lines were cloned as described in materials and methods. After several passages stable cell clones were established from both v-myc and egfr-neu - containing cell lines. These clones were characterised for the same markers as the cell lines of origin. The results for the clones originating from cell lines immortalised with the v-myc containing retroviral vector are shown in table 1. All clones showed a prominent expression of nestin and vimentin, some of the cells were GFAP- or LB1- positive.

The characterisation of the clones originating from the cell lines immortalised with the egfr-neu -expressing retroviral vector is shown in table 4.

### Induction of differentiation of the immortalised cell lines and clones

To induce differentiation in vitro several combinations of the following factors were used: retinoic acid (RA, 10 µM), NGF, β-FGF, EGF, IL-7, TGFα, dbcAMP (1mM). In addition the effect of co-culture with cortical glial cells originating from postnatal day 0 to 1 and granule neurons originating from postnatal day 6 mice was investigated.

### Differentiation of v-myc-expressing lines and clones

1mM dbcAMP induced a partial differentiation of the cells. The cells showed a more differentiated morphology, the expression of the markers vimentin and nestin was reduced and especially the cells of the cell line HNMMCV11.2. showed in addition to these changes an induction of the expression of MAP-2, a neuronal characteristic (table 3).

**Table 3**

| antigenic profile of v-myc expressing cell lines and clones after treatment | | | | | | | | | | | | |
|---|---|---|---|---|---|---|---|---|---|---|---|---|
| | Antibodies | | | | | | | | | | | |
| | A2B5 | LB1 | O4 | L1 | GFAP | NS | VIM | NF-P | tau | Synaptp. | NSE | MAP-2 |
| | | | | | | | | | | | | |

| cell lines + dbc AMP | | | | | | | | | | | | |
|---|---|---|---|---|---|---|---|---|---|---|---|---|
| 13.1 | + | - | - | - | + | +++ | +++ | | | | | |
| 14.1 | + | + | - | - | ++ | +++ | +++ | | | | | |
| 11.2 | - | + | + | - | ++ | +++ | +++ | | | | | ++ |

| cell clones + dbc AMP | | | | | | | | | | | | |
|---|---|---|---|---|---|---|---|---|---|---|---|---|
| 13.1.E | | ++ | - | - | +++ | +++ | +++ | - | - | - | | - |
| 13.1.F | | - | - | - | ++ | + | + | - | - | - | - | - |

| cell clone + neurons | | | | | | | | | | | | |
|---|---|---|---|---|---|---|---|---|---|---|---|---|
| 13.1.E | | + | - | - | ++ | +++ | +++ | - | - | - | | - |

**Table 4**

| antigenic profile of egfr-neu expressing cell line and clone cultured in absence of EGL (-EGL) | | | | | | | | | | | |
|---|---|---|---|---|---|---|---|---|---|---|---|
| | Antibodies | | | | | | | | | | |
| | A2B5 | LB1 | O4 | L1 | GFAP | NS | VIM | NF-P | tau | Synaptp. | MAP-2 |
| cell line: | | | | | | | | | | | |
| HC e/n XV | | | | | | | | | | | |
| 4 DIV - EGF | +++ | +++ | + | - | + | +++ | +++ | n.d. | n.d. | n.d. | ++ |

| cell clone: | | | | | | | | | | | |
|---|---|---|---|---|---|---|---|---|---|---|---|
| HC e/n XV B | | | | | | | | | | | |
| 4 DIV - EGF | n.d. | + | - | - | - | +++ | +++ | (+) | - | - | n.d. |
| 9 DIV-EGF | n.d. | + | + | - | ++ | +++ | +++ | (+) | - | - | ++ |

### Differentiation of egfr-neu expressing lines and clones

In the absence of EGF, where the activity of the oncogene is down-regulated, the cells of the lines showed a pronounced morphological differentiation. Many of the cells expressed highly elaborated branched processes in a neuronal- or in a few cases a oligodendrocyte-typical manner. As judged by the marker profile, the cell line HC egfr-neu XV expresses neuronal properties (table 4).

### Ca²⁺-imaging experiments

The cells were loaded with Fura 2/AM between day 3 and 7 in culture. A number of agonists were tested for their ability to activate specific receptors on the cell surface which leads to a rise in intracellular calcium. The rise in calcium concentration in the cytoplasm can occur after activation of specific calcium channels in the cell membrane, after activation of neurotransmitter receptors directly coupled to ion channels that are permeable to calcium ions (i.e. NMDA or AMPA type glutamate receptors), or by activation of receptors, which propagate the signal through the activation of specific G-proteins, which regulate the activity of different enzymes in the interior of the cell (Connor, Cell Calcium 14 (1993), 185-200; Crouch and Hendry, Medical Res. Rev. 13 (1993), 105-123). The activation of phospholipase C generates the second messengers diacylglycerol and inositoltriphosphate from phospholipids in the cell membrane. The binding of inositoltriphosphate to receptors localised on internal vesicles which store calcium ions leads to a transient rise in the concentration of calcium ions in the cytoplasm (Berridge, M.J. 1993, Nature 361: 315-325).

The cells were incubated with a number of agonists with different specificities for a variety of neurotransmitter receptors in the central nervous system. In particular specific ligands against different subtypes of glutamate receptors were tested. Glutamate and aspartate are believed to be the major excitatory neurotransmitters in the central nervous system of mammals (Nicholls, Eur.J.Biochem. 212 (1993) 613-631). The receptors for glutamate can be divided into receptors, which are directly coupled to cation-gated ion channels (the ionotropic receptors) and into receptors, which are coupled to G-proteins (the metabotropic receptors) and which activate second messenger systems (Hollmann et al., Nature 342 (1989), 643-648; Sommer et al., Science 249 (1990), 1580-1585; Pin, J.P. and Duvoisin,R. 1995, Neuropharmacology 34: 1-26).

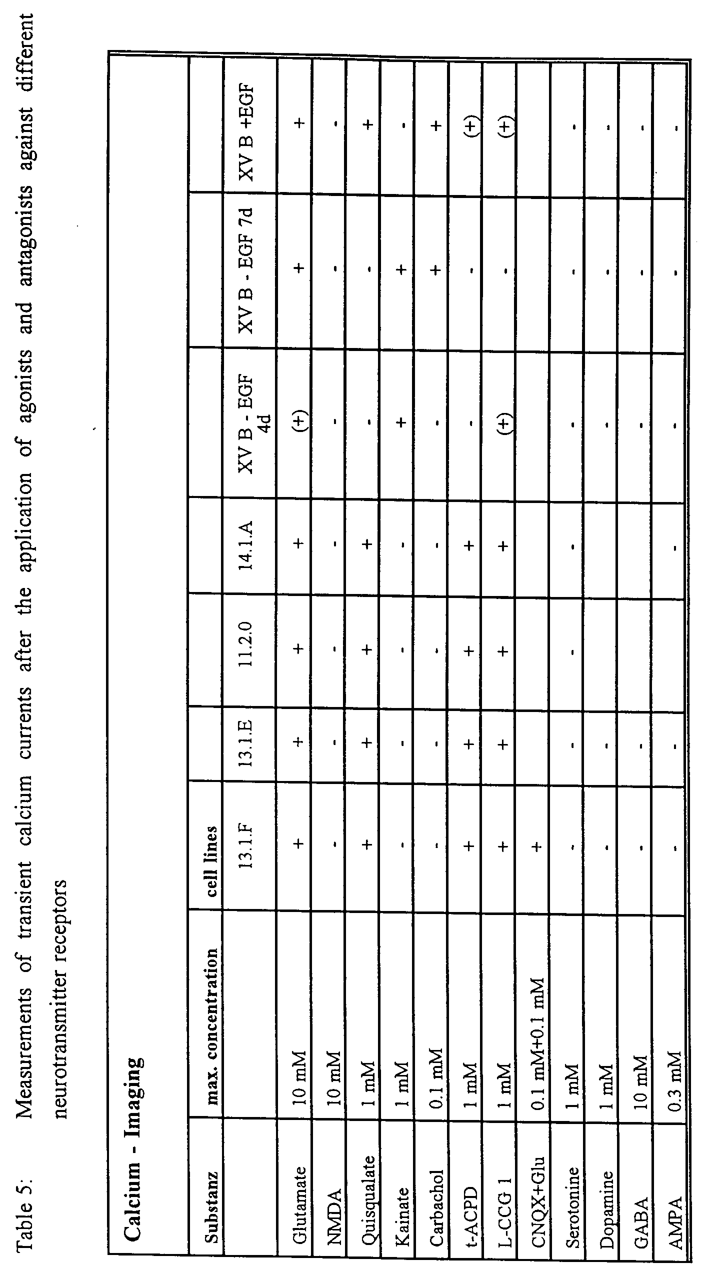

When agonists to different glutamate receptor subtypes were incubated with the clones and lines mentioned above, different signals were recorded (table 5). The application of glutamate from 10µM to 10 mM to the cell clones HNMMCV13.1.F, HNMMCV13.1.E, HNMMCV11.2.0, or HNMMCV14.1.A resulted in a significant rise in calcium concentration in the cytoplasm. Agonists, which are able to activate ionotropic glutamate receptors such as ∝-amino-3-hydroxy-5-methyl-4-isoxazol-propionic acid (AMPA, up to 1mM) or kainic acid (up to 1mM) did not result in a transient rise in intracellular calcium concentrations in the cell clones HNMMCV13.1.F, HNMMCV13.1.E, HNMMCV11.2.0, or HNMMCV14.1.A and in undifferentiated HC e/n XV.B cells with one exception: in the presence of kainate there was a reproducible calcium signal in undifferentiated HC e/n XV.B cells. The signals recorded after application of glutamate (100µM) was not antagonised by 6-nitro-7-cyanochinoxalin-2,3-dione (CNQX, 100µM), which selectively blocks ionotropic glutamate receptors. N-methyl-D-aspartate (NMDA; up to 500µM), which selectively activates another ionotropic subclass of glutamate receptors with calcium conducting properties (Kutsuwada, Nature 358 (1992), 36-41), did not result in significant calcium signals in these cells. The fast inactivating signals measured with electrophysiological recordings may not be visible with this system. From these experiments (with the exception of undifferentiated HC e/n XV.B cells for kainate) an expression of ionotropic glutamate receptors seems unlikely. Agonists to other subclasses of glutamate receptors, the metabotropic glutamate receptors (Tanabe, Neuron 8 (1992), 169-179), have also been tested for their ability to activate transient rises in intracellular calcium concentrations. After the activation of a metabotropic glutamate receptor and the activation of the corresponding G-protein this transient rise may occur through the activation of phospholipase C in the cell membrane (Bockaert, Fund.Clin.Pharmacol. 7 (1993), 473-485). Agonists to metabotropic glutamate receptors such as 1S3R-aminocyclopentane-1,3-dicarboxylate (1S3R-ACPD) and L-carboxycyclopropylglycin (L-CCG1) both resulted in strong (weak signals in differentiated HC e/n XV.B cells) and transiently occuring increases in the calcium concentration in the cells (table 5). These results suggest that the HNMMCV13.1.E, HNMMCV13.1.F, HNMMCV11.2.0, HNMMCV14.1.A and to a lesser extent the differentiated HC e/n XV.B cells seem to express glutamate receptors which correspond to the metabotropic, not to the ionotropic type of receptors.

A number of specific agonists for other neurotransmitter receptors have been applied to the cells. Serotonine (agonist for different serotonine neurotransmitter receptors (Perontka 1993, J.Neurochem. 60: 408-416), up to 1mM) and dopamine (specific for different dopamine neurotransmitter receptors Sibley 1992, Trends Pharmacol. Sci. 13: 61.69), up to 1mM) did not lead to a specific signal in 13.1.F, 13.1.E, 11.2.0, 14.1.A or HC e/n XV.B cells (table 5). Carbachol (specific for G-protein coupled muscarinic type of acetylcholine receptors (Milligan 1994, Trends Pharmacol. Sci. 14: 413-418), up to 1mM) showed a reproducible signal in HC e/n XV.B cells, but not in any other cell line tested.

### Second messenger experiments

The pharmacology of the metabotropic glutamate receptor(s) (mGluR) expressed by HNMMCV14.1A cells was characterized. The effect of several known agonists of mGluR on inositolphosphate accumulation was tested. L-glutamate, quisqualate, ibotenate and 1S,3R-ACPD caused a concentration dependent increase in inositolphosphate accumulation. D-glutamate was without effect. From the dose-response relationship the EC50 values were calculated to be 50µM (L-glutamate), 20nM (quisqualate), 5µM (ibotenate) and 20µM (1S,3R-ACPD), respectively. These data resulted in the following rank order of potency: Qa>Ibo>1S,3R-ACPD>Glu. L-glutamate was the most potent agonist. The maximum responses of ibotenate, 1S,3R-ACPD and quisqualate were slightly weaker than those observed with glutamate.

### Electrophysiology

**Ligand activated currents:** A number of specific agonists with different preferences for a variety of excitatory and inhibitory neurotransmitter receptors in the central nervous system were investigated. Although there was a variety of considerable transmitter specificity and sensitivity among cell lines, a general simplified statement can be made: Cells with sodium currents react to a variety of substances with large ligand activated currents. Cells without sodium currents tend to have smaller responses to fewer agonists (table 6).

**Table 6**

| electrophysiological recordings of voltage- and ligand-activated currents | | | | |
|---|---|---|---|---|
| | **13.1.E** | **14.1.A** | **XV. B** | **XV. B/ - EGF** |
| Sodium currents | + | - | - | ++ |
| Delayed rectifier | + | + | + / - | ++ |
| Inward rectifier | + / - | ++ | + | + / - |
| Glutamate | + | + / - | - | ++ |
| Kainate | n.t. | + / - | + | +++ |
| AMPA | n.t. | n.t. | n.t. | + |
| NMDA | n.t. | + / - | + / - | ++ |
| GABA | n.t. | ++ | + | +++ |
| Acetylcholine | n.t. | + | + / - | - |

The pharmacological and electrophysiological properties of the ligand activated currents induced by different agonists were in detail analysed in the cell clone which gave responses of large amplitude, namely differentiated HC e/n XVB cells. As for the calcium-imaging experiments different glutamate agonists induced distinct inward currents in HC e/n XV.B cells. AMPA (100µM), NMDA (1mM), kainate (1mM) and glutamate (1mM) evoked responses with different peak amplitudes. Some cells did not respond to NMDA and/or AMPA. In contrast, application of glutamate to HNMMCV13.1.E, HNMMCV14.1.A and undifferentiated HC e/n XVB cells induced only small inward currents. To identify the ionic species mediating the current activated by kainate and to elucidate the subunit composition of the presumable kainate/AMPA receptor the reversal potential of the kainate response was determined. The membrane was therefore depolarized or hyperpolarized to -100, -80, -60, -40, -20, 0, +20 and +40. The resulting I/V curve for kainate was linear and reversed at 0mV, indicating the presence of the GluR-B subunit, which is responsible for the linear current/voltage relationship.

Among the ionotropic transmitter receptors, GABA is the main inhibitory transmitter in the central nervous system. As for glutamate receptors, GABA receptors can be divided into ionotropic receptors (so called GABA_{A} receptors) and metabotropic receptors (GABA_{B} receptors). The GABA-response of HC e/n XVB cells could be mimicked by the specific GABA_{A} receptor agonist muscimol and blocked by the antagonist bicuculline. The reversal potential of the GABA-activated current was close to +10 mV, a value compatible with the predicted Cl- equilibrium potential. These experiments demonstrate that HC e/n XVB cells exhibit prominent resonses to GABA, mediated by GABA_{A}-receptors. In contrast to glutamate these receptors are also present on some undifferentiated HC e/n XVB cells and HNMMCV14.1.A cells. However, there were big fluctuations among the peak amplitudes of the GABA-responses of these cells and many cells with miniature GABA-responses.

Finally, after the application of acetylcholine only a minority of HNMMCV14.1.A and undifferentiated HC e/n XVB cells showed acetylcholine induced inward currents.

**Voltage activated currents:** In order to check for the presence or absence of voltage activated currents, the patched cells were clamped to a series of de- and hyperpolarising potentials starting from -70 mV, using a voltage clamp protocol. Depolarisation of all kind of cells activated an outward current component that displayed a delayed increase during the voltage step. However, the differentiated HC e/n XV.B cells often displayed an additional fast activating and inactivating inward current (table 6), presumably a sodium current, enabling them to generate action potentials under current clamp conditions. This current was not observed in cells from the cell lines HNMMCV14.1.A and undifferentiated HC e/n XVB cells. Hyperpolarisation to potentials more negative than -80 mV activated an extra inward current component. This inwardly rectifying current inactivated at more negative potentials and was less pronounced in cells having a sodium inward current.

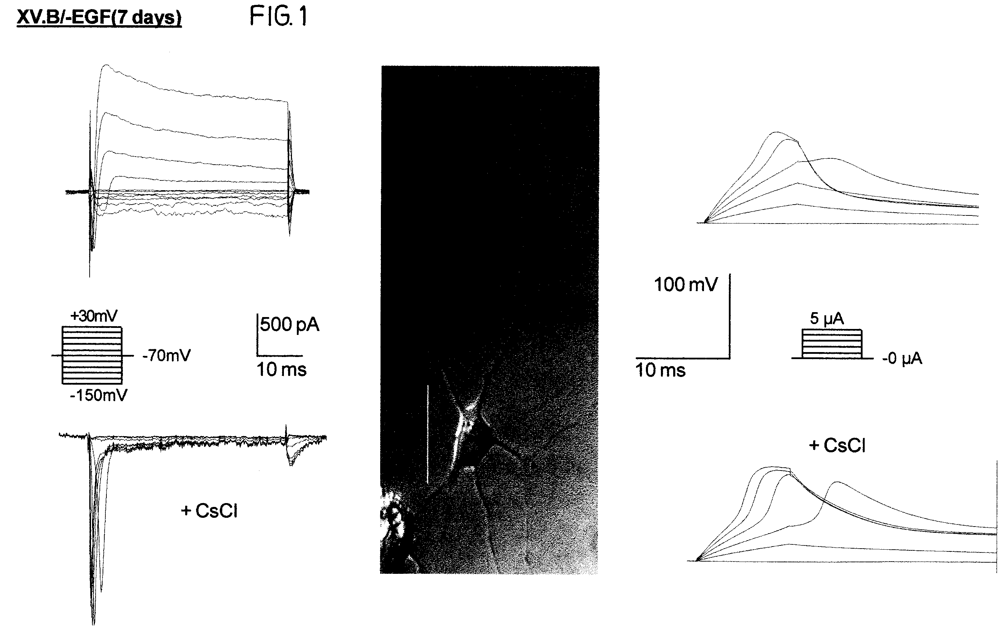

### Legend to Fig. 1:

Differentiated XV.B cells 7 days after EGF removal. On the left, voltage clamp recordings of two different cells with and without CsCl in the pipette solution. Using a voltage clamp protocol the mebrane was clamped from a holding potential of -70mV to increasing de- and hyperpolarising potentials. In the absence of CsCl the cells responded to large depolarisations with the activation of a fast sodium current and a delayed outward rectifying potassium current, which is blocked in the presence of CsCl. On the right corresponding cells were current clamped. At appropiate current injections action potentials were elicited. Note the extended repolarisation in the presence of CsCl. In the middle a typical differentiated XV.B cell is shown.

### Table legend:

### Description of the symbols and abbreviations used in the tables:

### antibodies recognising the following antigens:

**A2B5:** surface ganglioside expressed by glial precursor cells, some astrocytes and neurons
**LB1:** surface lipid on oligodendrocytes, late stage of glial precursor cells and Schwann cells
**O4:** surface molecules on oligodendrocytes, late stage of glial precursor cells and Schwann cells
**L1:** surface glycoprotein expressed by neurons and Schwann cells
**GFAP:** glial fibrillary acidic protein; intracellular protein expressed by astrocytes
**NS:** nestin; intracellular protein expressed by neural precursor cells
**VIM:** vimentin; intracellular protein expressed by neural precursor cells
**NF-160:** 160 kD form of neurofilament; intracellular protein expressed by neurons
**NF-P:** early phosphorylated form of the high molecular weight kD form of neurofilament; intracellular protein expressed by neurons
**tau:** microtubule-associated protein tau
**synapto:** Synaptophysin
**NSE:** neuron specific enolase
**MAP-2:** microtubule-associated-protein 2

### further abbreviations:

**DIV:** days in vitro
**+dbcAMP:** cultured in presence of 1 mM dibutyryl cyclic adenosine monophosphate
**+ or - EGF:** cultured in presence or absence of epidermal growth factor
**+ neurons:** co-cultured with cerebellar granule cells
**NGF:** Nerve Growth Factor
**β-FGF:** basic Fibroblast Growth Factor
**EGF:** Epidermal Growth Factor
**n.d.:** not done

### Key:

- (+):: 1-10% positive cells
- +:: 10-30% positive cells
- ++:: 30-70% positive cells
- +++:: 70-100% positive cells
- -:: no positive cells

## Claims

1. Immortalized hippocampal cells expressing metabotropic glutamate receptors.

2. Cells according to claim 1, deposited with the Deutsche Sammlung für Mikroorganismen under designations ACC 2223, ACC 2230, ACC 2231, ACC 2232 and ACC 2233.

3. Use of the cells according to claims 1 and 2 for screening of medicaments.
